**Europäisches Patentamt**

**European Patent Office**

**Office européen des brevets**

(19)

(11) Veröffentlichungsnummer : **0 030 000**
**B1**

(12)

# EUROPÄISCHE PATENTSCHRIFT

(45) Veröffentlichungstag der Patentschrift :
**16.11.83**

(51) Int. Cl.³ : **B 65 B 53/06**

(21) Anmeldenummer : **80107385.9**

(22) Anmeldetag : **26.11.80**

(54) **Vorrichtung zum Schrumpfen von insbesondere palettierten und mit einer Schrumpfhaube überzogenen Stapeln.**

(30) Priorität : **01.12.79 DE 2948519**

(43) Veröffentlichungstag der Anmeldung :
**10.06.81 Patentblatt 81/23**

(45) Bekanntmachung des Hinweises auf die Patenterteilung : **16.11.83 Patentblatt 83/46**

(84) Benannte Vertragsstaaten :
**AT BE CH DE FR GB IT LI LU NL SE**

(56) Entgegenhaltungen :
**DE A 1 776 007**
**DE A 2 616 202**
**FR A 2 158 936**

(73) Patentinhaber : **MSK-Verpackungs-Systeme Gesellschaft mit beschränkter Haftung**
**Benzstrasse Postfach 1610**
**D-4190 Kleve (DE)**

(72) Erfinder : **Hannen, Rainer Wilhelm**
**Nachtigallenweg 6**
**D-4180 Goch 2 (DE)**

(74) Vertreter : **Stark, Walter, Dr.-Ing.**
**Moerser Strasse 140**
**D-4150 Krefeld (DE)**

EP 0 030 000 B1

Vorrichtung zum Schrumpfen von insbesondere palettierten und mit einer Schrumpfhaube überzogenen Stapeln

Die Erfindung betrifft eine Vorrichtung zum Schrumpfen von insbesondere palettierten und mit einer Schrumpfhaube überzogenen Stapeln, bestehend aus einer Brenneranordnung und zugeordneter Zünd- und Kontrolleinrichtung sowie einem Kasten, dessen Boden eine Stellfläche für Gasflaschen bildet.

Derartige Vorrichtungen sind als stationäre Vorrichtung oder auf einem Karren montiert bekannt (DE-A-21 53 001). In allen Fällen, bei denen zur Gasversorgung der Brenner Gasflaschen mit Flüssiggas, insbesondere Propangas vorgesehen sind, d. h. insbesondere bei fahrbaren Vorrichtungen, besteht die Gefahr, daß wegen der verhältnismäßig großen Gasentnahme aus den Gasflaschen diese oder deren zugeordnete Ventilanordnungen insbesondere dann vereisen, wenn die Vorrichtung im Freien verwendet wird. Dann spricht der Gasdruck zusammen und ein weiteres Arbeiten ist nicht möglich. Die Vereisung kann bereits nach wenigen Betriebsminuten eintreten, so daß es in der Regel nicht möglich ist, mehrere Schrumpfeinheiten unmittelbar nacheinander zu behandeln.

Zum Ausgleich des Wärmeverlustes muß den Gasflaschen die durch Verdampfungswärme entzogene Wärme wieder zugeführt werden. Dabei muß allerdings beachtet werden, daß die Gasflaschen sich nicht überhitzen dürfen, weil sonst Explosionsgefahr besteht.

Aufgabe der Erfindung ist es, mit einer Vorrichtung der eingangs beschriebenen Gattung einen durchgehenden Betrieb zu ermöglichen und insbesondere den Wärmeverlust der Gasflaschen auszugleichen.

Diese Aufgabe wird mit einer Vorrichtung der eingangs beschriebenen Gattung gelöst, dadurch gekennzeichnet, daß der Kasten wenigstens teilweise mit Wasser gefüllt ist, daß die dem Stapel zugekehrte Seitenwandung des Kastens außen mehrere Wärmetauscherrippen zur Aufnahme der vom Stapel reflektierten Strahlungswärme aufweist und daß die Seitenwandung mit den Wärmetauscherrippen sich über eine größere Höhe als der Kasten erstreckt, wobei der sich unter den Kasten erstreckende Teil der Seitenwandung doppelwandig ist und einen Wasserbehälter bildet, der mit dem Inneren des Kastens in Verbindung steht.

Die Verwendung von Wasser als Heizmedium für die von Vereisung bedrohten Gasflaschen stellt zunächst einmal sicher, daß die Gasflaschen nicht überhitz werden können. Andererseits bestehen einfache Möglichkeiten, die Erwärmung des Wassers so einzurichten, daß die über das erwärmte Wasser den Gasglaschen zugeführte Wärme im Wesentlichen der durch Verdampfungswärme entzogenen Wärme entspricht. Dafür genügt es bereits, wenn die Höhe des Kastens etwa 1/3 bis 1/2 der Höhe der Gasflaschen beträgt und die Gasflaschen dementsprechend nur mit ihrem unteren Teil in das Wasser gesetzt sind. Durch Kopplung der dem Wasser zugeführten Wärme an die von der Brenneranordnung abgegebene Leistung und damit an die jeweilige Entnahme aus den Gasflaschen gelingt es dann, den Gasflaschen im wesentlichen diejenige Wärmemenge wieder zuzuführen, die durch Verdampfung des Flüssiggases verlorengegangen ist. Einer besonderen Regelung bedarf es dann nicht.

Das Wasser wird dadurch aufgeheizt, daß die dem Stapel zugekehrte Seitenwandung des Kastens außen mehrere Wärmetauscherripen zur Aufnahme der vom Stapel reflektierten Strahlungswärme aufweist. Diese Strahlungswärme kann ganz erhebliche Beträge annehmen und wird auf diese Weise günstig ausgenutzt, in dem die Wärmetauscherrippen als Übertragungselemente funktionieren.

Insbesondere kann die Seitenwandung mit den Wärmetauscherrippen sich über eine größere Höhe als der Kasten erstrecken, wobei der sich unter den Kasten erstreckende Teil der Seitenwandung doppelwandig ist und einen Wasserbehälter bildet, der mit dem Inneren des Kastens in Verbindung steht. Auf diese Weise kann ein verhältnismäßig großer Teil der Strahlungswärme genutzt werden. Im Wasserbehälter bzw. im Kasten strömt das Wasser wiederum unter Konvektionswirkung. Als Material für den Kasten und ggf. die Wärmetauscherrippen empfiehlt sich ein gut wärmeleitendes Material, insbesondere Aluminium.

Im Folgenden wird ein in der Zeichnung dargestelltes Ausführungsbeispiel der Erfindung erläutert ; es zeigen :

Figur 1 eine Draufsicht auf eine Vorrichtung zum Schrumpfen von palettierten Gutstapeln, die mit einer Schrumpfhaube überzogen sind,

Figur 2 eine Seitenansicht des Gegenstandes nach Fig. 1,

Figur 3 teilweise eine Draufsicht auf eine andere Ausführungsform des Gegenstandes nach Fig. 1,

Figur 4 einen Schnitt in Richtung IV-IV durch den Gegenstand nach Fig. 3,

Figur 5 in Draufsicht und teilweise geschnitten einen Teil des Gegenstandes nach Fig. 1,

Figur 6 einen Schnitt in Richtung VI-VI durch den Gegenstand nach Fig. 5.

Mit der dargestellten Vorrichtung sollen Schrumpfhauben auf palettierten Gutstapeln 1 geschrumpft werden. Dazu besteht die Vorrichtung aus einem dreirädrigen Karren 2, auf dem sich eine Brenneranordnung 3, zwei Gasflaschen 4 sowie eine Zünd- und Kontrolleinrichtung befinden. Zwei Räder 5, 6 des Karrens 2 laufen auf einer gemeinsamen Radachse 7, während das dritte Rad 8 unter einem sich in Fahrtrichtung 9 von der Radachse 7 erstreckenden Träger 10 um eine vertikale Achse drehbar als Nachlaufrad angeordnet ist. Eine in Fahrtrichtung 9 hinter der Radachse 7 angeordnete Deichsel 11 mit zwei Handgriffen 12, 13 dient zum Schieben des Karrens 2, der, wie ohne weiteres ersichtlich, wegen des freibeweglichen Nachlaufrades 8, auch steuerbar ist.

Damit der Karren 8 und mit ihm die Brenneranordnung 3 in einem vorbestimmten Abstand längs der zu behandelnden Seite des Gutstapels verfahren werden kann, ist am Träger 10 im Bereich der vertikalen Drehachse des Nachlaufrades 8 ein Kragarm 14 angeschlossen, der eine um eine vertikale Achse 15 frei drehbare Tastrolle 16 trägt. Die Tastrolle weist zweckmäßig einen verhältnismäßig großen Durchmesser auf, der jedenfalls größer sein sollte, als die halbe Karrenbreite. Die Tastrolle 16 kann relativ zum Träger 10 auch verstellbar angeordnet sein, so daß der Abstand der Brenneranordnung 3 von der zu behandelnden Seite des Gutstapels 1 einstellbar ist. Das ist jedoch in der Zeichnung nicht dargestellt. Auf jeden Fall ermöglicht die Anordnung des Nachlaufrades 8 zusammen mit der Tastrolle 16 eine sehr genaue Führung des Karrens 2 bzw. der Brenneranordnung 3 relativ zu der zu behandelnden Seite des Gutstapels 1. Besonders günstige Verhältnisse werden dann erreicht, wenn die vertikale Achse 15 der Tastrolle 16 den gleichen Abstand von der Radachse 7 hat wie die vertikale Schwenkachse des Nachlaufrades 8.

Beim dargestellten Ausführungsbeispiel sind zwei Gasflaschen 4 nebeneinander direkt oberhalb der Radachse 7 angeordnet. Das hat den Vorteil, daß die Belastung des Nachlaufrades 8 sich während des Betriebes, d. h. im Zuge der Entleerung der Gasflaschen 4, nicht ändert. Im übrigen ist die Gewichtsverteilung des Karrens 2 insgesamt so ausgelegt, daß das Nachlaufrad 8 nur verhältnismäßig wenig, vorzugsweise mit ca. 15 kg belastet ist, um die Steuerfähigkeit des Karrens 2 nicht zu beeinträchtigen.

In der Zeichnung ist nicht dargestellt, daß in Fahrtrichtung 9 hinter der Radachse 7 eine weitere Stütze angeordnet sein kann, die, sofern es sich um eine starre Stütze handelt, während des Betriebes genügend Bodenfreiheit zuläßt. Die Stütze kann aber auch als Nachlaufrad ausgebildet sein, das auf dem Boden abgestützt ist.

Wenn ein gleichmäßiger Schrumpf der Schrumpfhaube am Gutstapel 1 erzielt werden soll, ist es nicht nur wichtig, den Abstand der Brenneranordnung 3 vom Gutstapel während der Bewegung des Karrens 2 am Gutstapel vorbei bzw. um den Gutstapel herum einzuhalten, sondern auch eine vorgegebene Geschwindigkeit des Karrens 2 einzuhalten. Dazu kann am Karren an einer für die Bedienungsperson gut sichtbaren Stelle eine Geschwindigkeitsanzeige angebracht sein, die ggf. auch optische oder akustische Warnsignale liefert, wenn ein vorgegebener Geschwindigkeitsbereich über- oder unterschritten wird. Es versteht sich, daß eine Einstellung auf eine vorgegebene Geschwindigkeit möglich sein sollte.

Um zu verhindern, daß die Gasflaschen 4 bzw. deren zugeordnete Ventilanordnungen während des Betriebes durch den bei Verdampfung des Flüssiggases auftretenden Wärmeentzug vereisen, sind die Gasflaschen in einem oberhalb der Radachse 7 angeordneten Kasten 17 aufgestellt, der beheizbares Wasser enthält. Der Kasten 17 ist so weit mit Wasser gefüllt, daß der Wasserspiegel 18 sich etwa auf 1/3 der Höhe des Kastens 17 befindet und dementsprechend lediglich der untere Teil der Gasflaschen 4 im Wasser steht. Das reicht im allgemeinen aus, um eine Vereisung zu verhindern.

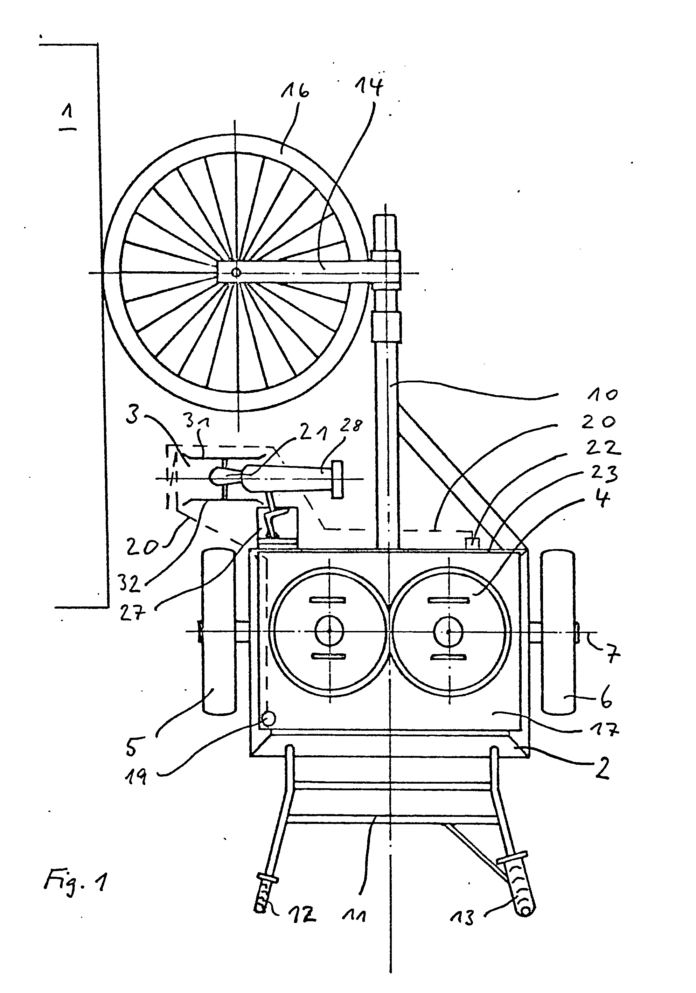

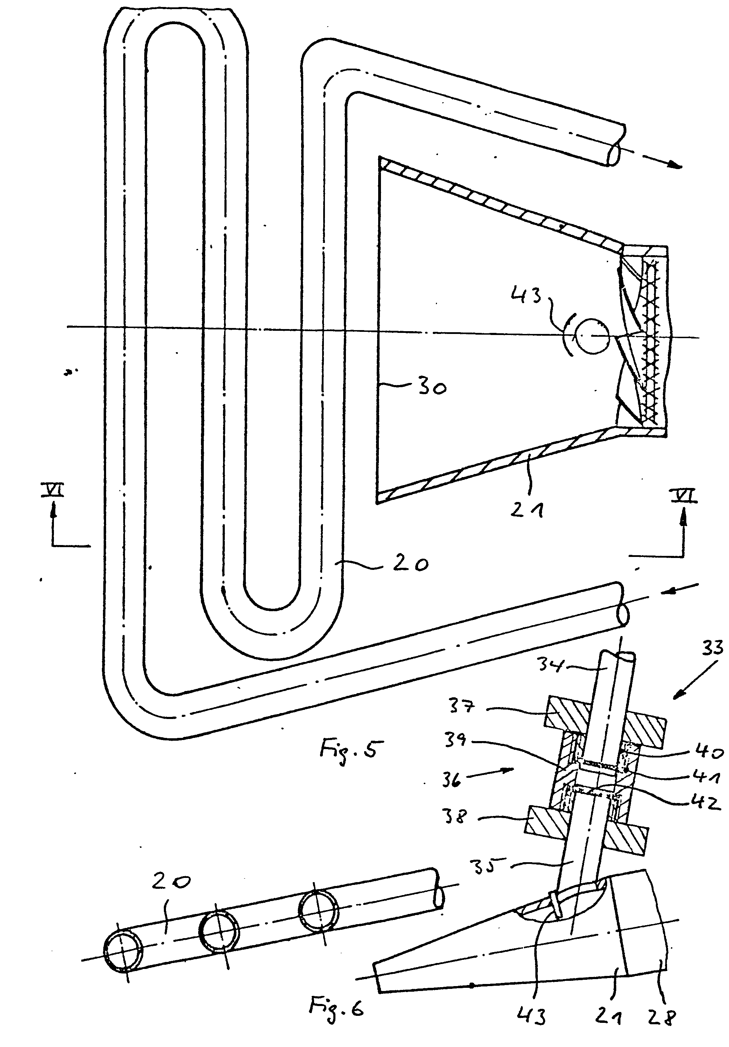

Bei dem in den Figuren 1 und 5 dargestellten Ausführungsbeispiel weist der Kasten 17 bodenseitig einen Anschluß 19 für eine Rohrschlange 20 auf, die unter dem Boden des Kastens 17 bis vor die unterste Düse 21 der Brenneranordnung 3 und von dort zum Kasten 17 zurückgeführt ist, so daß die Rohrschlange 20 an einem Stutzen 22 der in Fahrtrichtung 9 vorn liegenden Seitenwandung 23 etwa in Höhe des Wasserspiegels 18 mündet. Der Anschluß 19 und der Stutzen 22 sind im Bereich gegenüberliegender Ecken des Kastens 17 angeordnet, so daß das erwärmte Wasser, welches sich konvektiv durch die Rohrschlange 20 bewegt, nach dem Austritt aus dem Stutzen 22 beide Gasflaschen 4 umspülen kann.

Da die Erwärmung des Wassers von der jeweiligen Brennerleistung abhängt und die Brennerleistung wiederum von der Entnahme aus den Gasflaschen 4, wird der Wärmeverlust der Gasflaschen 4 durch Verdampfung weitgehend selbsttätig ausgeglichen.

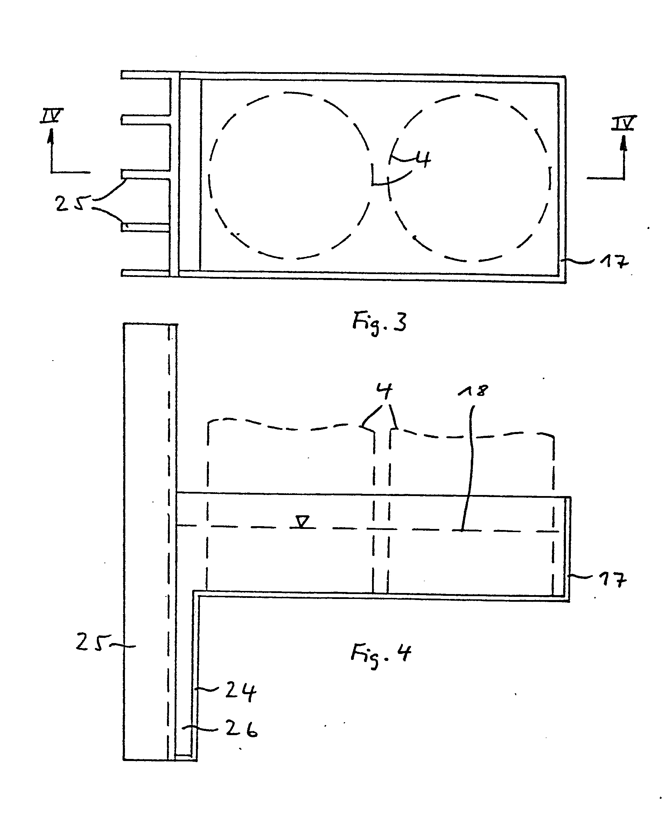

Bei einer, in den Figuren 3 und 4 dargestellten Ausführungsform des Kastens 17 weist die dem Gutstapel 1 zugekehrte Seitenwandung 24 des Kastens 17 außen mehrere Wärmetauscherrippen 25 auf. Diese Wärmetauscherrippen 25, die ebenso wie der Kasten 17 aus einem gut wärmeleitenden Material, z. B. Aluminium bestehen, nehmen die vom Gutstapel 1 reflektierte Strahlungswärme auf und geben sie an das im Kasten 17 enthaltene Wasser ab. Um die Ausnutzung der Strahlungswärme und den Wärmeübergang zu verbessern, erstrecken sich die Wärmetauscherrippen 25 oben und unten über den Kasten 17 hinaus. Auch die dem Gutstapel 1 zugekehrte Seitenwandung 25 des Kastens 17 erstreckt sich bis unter den Kasten 17, wobei zwischen den Wärmetauscherrippen 25 und dieser Seitenwandung 24 ein Wasserbehälter 26 gebildet ist, wenn dessen Wasservorrat mit dem Wasservorrat des Kastens 1 in Verbindung steht. Auch bei dieser Ausführungsform erfolgt der Transport des aufgeheizten Wassers durch Konvektion, wobei die Aufheizung des Wassers nach Maßgabe der vorhandenen Brennerleistung erfolgt.

Eine weitere, in den Figuren nicht dargestellte Möglichkeit zur Aufheizung des Wassers im Kasten 17 besteht darin, daß z. B. unterhalb des Kastens 17 ein weiterer, nicht dargestellter Brenner angeordnet ist, der den Kasten 17 unmittelbar beheizt. Dieser Brenner sollte aber an die Zünd- und Kontrolleinrichtung der Brenneranordnung angeschlossen sein, damit die Aufheizung des Wassers beim Betrieb wiederum an die Leistung der Brenner bzw. an die Entnahme aus den Gasflaschen 4 gekoppelt ist.

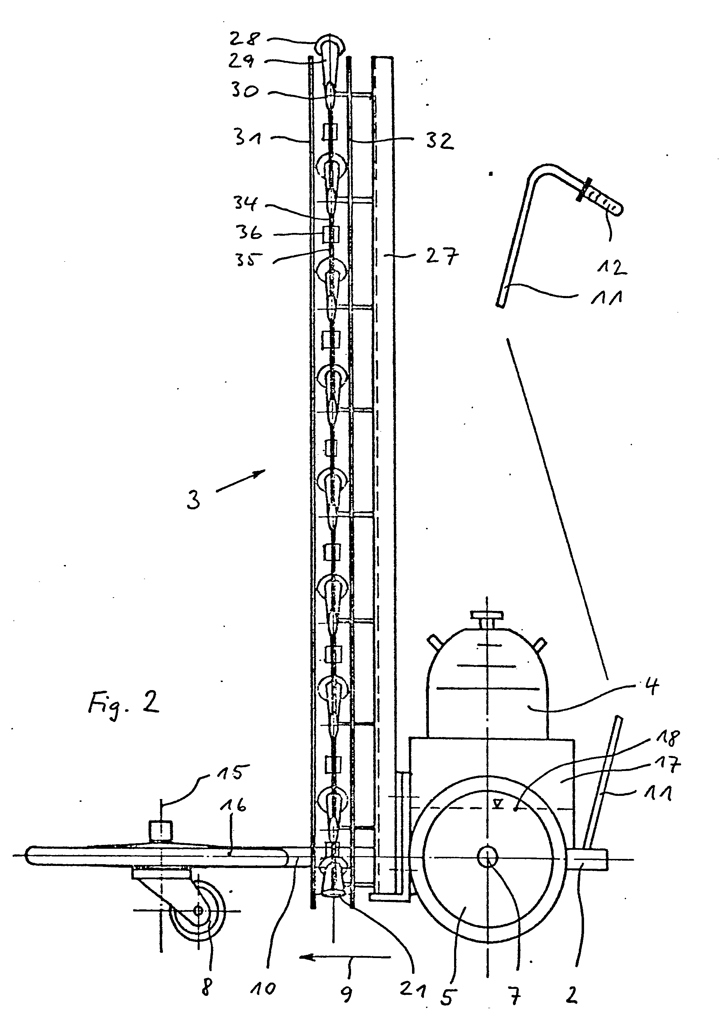

Die in den Figuren 2, 5 und 6 dargestellte Brenneranordnung 3 weist einen vertikalen Haltemast 27 mit mehreren daran übereinander angeordneten Brennern 28 auf. Jedem Brenner 28 ist eine Düse 29 zugeordnet. Die Düsen bestehen aus in Strömungsrichtung der Gase zunehmend flacher gedrückten Rohren und bilden eine ovale bis elyptische Düsenmündung 30.

Um eine günstige Verteilung der von der Brenneranordnung 3 & gegebenen Heizgase zu erreichen, sind die Düsenmündungen 30 der Düsen 29 mit Ausnahme der untersten Düse 21 so angeordnet, daß die Düsenmündungen sich mit ihrer Hauptachse im wesentlichen parallel zur Längsachse des Haltemastes 27 erstrecken. Die Düsenmündung 30 der untersten Düse 21 ist demgegenüber quer, d. h. horizontal angeordnet, weil diese Düse 21 das Heizgas unter den Gutstapel blasen soll, um den sogenannten Unterschrumpf zu erzeugen. Ggf. kann ded Brenner der untersten Düse 21 zu diesem Zweck und für die zusätzliche Aufheizung des Wassers stärker ausgelegt sein.

Aus Sicherheitsgründen sind beidseits der Reihe von Brennern 28 bzw. 21 Luftleitbleche 31, 32 angeordnet, die zur Kontrolle der Brenner durchsichtig sein können. Dementsprechend können die Luftleitbleche 31, 32 aus Gitterblechen oder aus einem durchsichtigen Werkstoff, z. B. durchsichtigem Kunststoff bestehen.

Die dargestellte Vorrichtung braucht grundsätzlich keine Hilfsenergiequelle z. B. eine Batterie mitzuführen, weil die Brenner mit einem piezoelektrischen Zünder, der im Handgriff 13 untergebracht ist, gezündet werden. Damit nicht jeder Brenner einzeln gezündet werden muß, wird mit dem Zünder im Handgriff 13 lediglich der unterste Brenner der Brenneranordnung 3 gezündet. Alle Brenner 28 sind untereinander durch Überzündrohre 33 verbunden, durch die ein Teil der gezündeten Gase in den nächsten Brenner übertritt und diesen zündet. Dadurch wird die Reihe der Brenner ausgehend vom untersten Brenner bis zum obersten Brenner in kürzester Zeit gezündet. Am obersten Brenner kann eine nicht dargestellte Kontrolleinrichtung vorgesehen sein, die den Betriebszustand dieses obersten Brenners und damit der Brenneranordnung 3 insgesamt überwacht. Es kann sich um eine elektronische oder um eine anders arbeitende Einrichtung handeln.

Die Überzündrohre 33 bestehen jeweils aus an die Düsen 21 bzw. 29 angeschlossenen Rohrabschnitten 34, 35, die jeweils durch Rohrkupplungen 36 miteinander verbunden sind. Jede Rohrkupplung 36 weist zwei auf die Enden der zugeordneten Rohrabschnitte 34, 35 gesetzte Überwurfmuttern 37 bzw. 38 sowie eine für den Anschluß dieser Überwurfmuttern 37, 38 eingerichtete Muffe 39 auf. Jede Überwurfmutter 37 bzw. 38 weist einen Axialflansch 40 mit Außengewinde auf, der in ein zugeordnetes Innengewinde einer Stufenbohrung 41 an der zugeordneten Anschlußseite der Muffe 39 eingedreht wird. Zwischen der Stirnseite des Axialflansches 40 und der Stufe der Stufenbohrung 41 wird ein auf das Ende des zugeordneten Rohrabschnittes 34 bzw. 35 aufgeschobener Dichtring 42 aus z. B. einem Kunststoffmaterial so eingeklemmt und verformt, daß er das Ende des zugeordneten Rohrabschnittes 34 bzw. 35 gegen die Stufenbohrung 41 abdichtet.

Wie man den Figuren 5 und 6 entnimmt, ist im Anschlußbereich der Rohrabschnitte 34 bzw. 35 innenseitig in der Düse jeweils ein Staublech 43 angeordnet, das in Strömungsrichtung der Gase vor der Anschlußöffnung für die Rohrabschnitte 34 bzw. 35 an der Innenwandung der Düse sitzt. Dieses Staublech 43 besitzt eine verhältnismäßig geringe Höhe, die jedenfalls kleiner ist als der Durchmesser der Rohrabschnitte 34, 35 und erstreckt sich kreisbogenförmig um die Öffnung für die Rohrabschnitte 34, 35 über eine Breite, die im wesentlichen dem Durchmesser dieser Rohrabschnitte entspricht. Das Staublech 43 sorgt zusammen mit der Verjüngung der Düse dafür, daß bei Zündung der zugeordneten Brenner die Flamme durch das entsprechende Überzündrohr 33 bis in den nächsten Brenner gelangt und diesen zündet. Zum Zünden der Brenneranordnung 3 genügt folglich die Betätigung des piezoelektrischen Zünders im Handgriff 13.

## Ansprüche

1. Vorrichtung zum Schrumpfen von insbesondere palettierten und mit einer Schrumpfhaube überzogenen Stapeln, bestehend aus einer Brenneranordnung und zugeordneter Zünd- und Kontrolleinrichtung sowie einem Kasten, dessen Boden eine Stellfläche für Gasflaschen bildet, dadurch gekennzeichnet, daß der Kasten (17) wenigstens teilweise mit Wasser gefüllt ist, daß die dem Stapel zugekehrte Seitenwandung (24) des Kastens (17) außen mehrere Wärmetauscherrippen (25) zur Aufnahme der vom Stapel reflektierten Strahlungswärme aufweist und daß die Seitenwandung (24) mit den Wärmetauscherrippen sich über eine größere Höhe als der Kasten (17) erstreckt, wobei der sich unter den Kasten (17) erstreckende Teil der Seitenwandung (24) doppelwandig ist und einen Wasserbehälter (26) bildet, der mit dem Inneren des Kastens (17) in Verbindung steht.

2. Vorrichtung nach Anspruch 1, dadurch gekennzeichnet, daß der Kasten (17) und die Wärmetauscherrippen (25) aus gut wärmeleitendem Material, insbesondere Aluminium, bestehen.

## Claims

1. A shrinking device, in particular for palletized stacks over which a shrinkable wrapping has been drawn, comprising a burner arrangement and an associated ignition and control device and a box, the base of which forms a positioning area for glass bottles, characterized in that the box (17) is at least partially filled with water, the lateral wall (24) of the box (17) facing the stack comprises a plurality of heat exchanger ribs (25) on the outside for absorbing the radiation heat reflected by the stack and the lateral wall (24) with the heat exchanger ribs extends over a greater height than the box (17), the part of the lateral wall (24) extending beneath the box (17) being double-walled and forming a water tank (26) which communicates with the interior of the box (17).

2. A device according to Claim 1, characterized in that the box (17) and the heat exchanger ribs (25) consist of good heatconducting material, in particular aluminium.

**Revendications**

1. Dispositif de rétraction, en particulier pour piles sur palettes enveloppées avec une housse rétractable, dispositif comprenant un ensemble de brûleurs auquel est affectée une installation d'allumage et de contrôle, ainsi qu'un caisson, dont le fond forme une surface de dépôt pour des bouteilles de gaz liquéfié, dispositif caractérisé en ce que le caisson (17) est rempli d'eau au moins en partie, en ce que la paroi latérale (24) du caisson (17), qui est tournée vers la pile est pourvue extérieurement de plusieurs nervures d'échange de chaleur (25) pour capter la chameur de rayonnement réfléchie par la pile, et en ce que cette paroi latérale (24), avec les nervures d'échange de chaleur s'étend sur une plus grande hauteur que le caisson (17), la partie de la paroi latérale (24), qui s'étend au-dessous du caisson (17), étant à double paroi et formant un réservoir d'eau (26), qui est en communication avec l'intérieur du caisson (17).

2. Dispositif suivant la revendication 1, caractérisé en ce que le caisson (17) et les nervures d'échange de chaleur (25) sont constitués en un matériau bien conducteur de la chaleur, en particulier en aluminium.

Fig. 1

Fig. 2

Fig. 3

Fig. 4

Fig.5

Fig.6